# EUROPEAN PATENT SPECIFICATION

(11) **EP 0 614 070 B1**
(45) Date of publication and mention of the grant of the patent: **17.06.1998**
(21) Application number: 94301068.6
(22) Date of filing: 14.02.1994
(51) Int. Cl.: G01C 22/02, A61B 5/024

(54) **An electronic pedometer with blood pulse counting device**
Elektronischer Pedometer mit einem Blutpulszähler
Pédomètre électronique avec un compteur d'impulsions sanguines

(30) Priority: 22.02.1993 JP 32310/93
(43) Date of publication of application: 07.09.1994
(73) Proprietor: SEIKO INSTRUMENTS INC., Tokyo 136 (JP)
(72) Inventor: Nakamura, Chiaki, Koto-ku, Tokyo (JP)
(74) Representative: Sturt, Clifford Mark

(56) References cited:
- US-A- 4 312 358
- US-A- 4 807 639
- US-A- 4 962 469
- US-A- 4 974 601
- PATENT ABSTRACTS OF JAPAN vol. 9, no. 253 (P-395) (1976) 11 October 1985 & JP-A-60 104 286 (SEIKO DENSHI KOGYO K.K.) 8 June 1985
- PATENT ABSTRACTS OF JAPAN vol. 5, no. 146 (P-80) (818) 16 September 1981 & JP-A-56 079 382 (MATSUSHITA DENKO K.K.) 29 June 1981

## Description

This invention relates to an electronic pedometer with pulse counting device which is equipped with a pulse counting function as well as a pedometer function. The pulse counting device is suitable for measuring the heart rate.

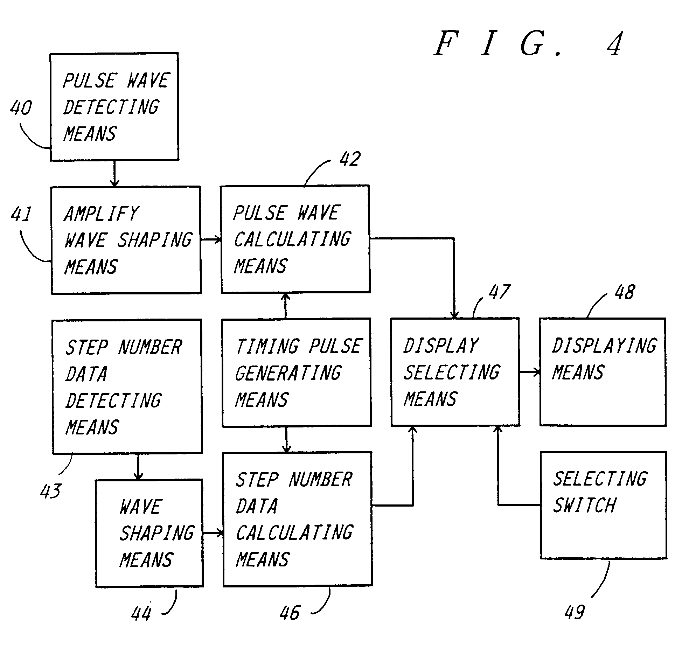

Fig. 4 is illustrative of a functional block diagram which shows the operations of an electronic pedometer with a pulse counting device in accordance with the prior art. Pulse wave detecting means 40 detects a pulse wave signal and outputs the detected signal to an amplify wave shaping means 41. The amplify wave shaping means 41 outputs to a pulse wave calculating means 42, a pulse wave signal which has its waveform shaped by the amplify wave shaping means 41. The pulse wave calculating means 42 calculates the cycle of the pulse wave signal input thereto based on a standard signal output by a timing pulse generating means 45. The pulse wave calculating means 42 outputs to a display selecting means 47, the pulse wave information which has been calculated. Step number data detecting means 43 detects a step signal and outputs to a wave shaping means 44, the step signal which has been detected. The wave shaping means 44 shapes the wave of the step signal which has been input and outputs to a step number data calculating means 46, the wave-shaped step signal. The step number data calculating means 46 calculates the cycle of the step signal which has been input based on a standard signal which is output from the timing pulse generating means 45. The step number data calculating means outputs into the display selecting means 47 the calculated step information. The display selecting means 47 above selects from the pulse wave information and step number information, both of which have been input to it, by referring to the output level which is output by a selecting switch 49, and outputs to a displaying means 48. The displaying means 48 displays the information which is input.

Such an electronic pedometer with pulse counting device as mentioned above is disclosed in Japanese Patent Publication No. JP-A-56-79382 (1981). In this electronic pedometer with pulse counting device, the pulse sensor by which a pulse wave and its number is measured and step number sensor which measures the number of steps are equipped separately. Thus the pulse wave measuring function and the step number measuring function can be used in the way that both functions are manually selected alternately by the selecting switch.

Increasingly, people who wish to do self health control through sporting activities, such as jogging. In these activities pulse measuring device and step number devices are widely used for gaining the information for self health control.

But the pedometer with the pulse counting device in the prior art has the demerit when designing smaller pedometers or portable one. In addition, such pedometers need more man-hours in the manufacturing process since both sensors of the pulse wave counting and step number counting or detecting are equipped separately. Finally, in its practical use, it is not easy to use, since the selecting switch has to be manually set.

An objection of this invention is to propose a handy, portable electronic pedometer with pulse counting device which is easy in practice to use.

According to the present invention, there is provided an electronic pedometer comprising:
a pulse wave detecting means for detecting the pulse wave of a mammal; and
pulse wave calculating means for amplifying an output signal from the pulse wave detecting means and for calculating the pulse rate and number of steps of the mammal;
characterised in that the pedometer further comprises pulse wave level storing means for storing historic data correlating type of movement with pulse wave characteristics of mammals;
action noise detecting means for comparing the signal received from the pulse wave detecting means with data stored in the pulse wave level storing means thereby detecting the type of movement of the mammal;
constant value storing means for storing historic data of an amplifying factor of the pulse wave calculating means; and
pulse wave calculate control means for selecting an amplifying factor of the pulse wave calculating means based on an output signal of the action noise detecting means and an output signal of the constant value storing means.

The invention is defined in Claims 1-7.

Embodiments of the present invention will now be described with reference to the accompanying drawings, of which:
Fig. 1 shows a functional block diagram of representative constitutions in this invention;
Fig. 2 shows another functional block diagram of representative constitutions in this invention;
Fig. 3 shows yet another functional block diagram of the representative constitutions in this invention;
Fig. 4 shows a functional block diagram of a pedometer with pulse count device in the prior art;
Fig. 5 shows an example of a displayed identified signal;
Fig. 6 shows an example of graphical display;
Fig. 7 shows a functional block diagram in a first embodiment of the pedometer with pulse count device in this invention;
Fig. 8 shows a functional block diagram in another embodiment of the pedometer with pulse count device in this invention;
Fig. 9 shows a circuit drawing of an embodiment of the pedometer with pulse count device in this invention;
Fig. 10 shows a flow chart of an amplifying ratio setting procedure in a CPU in an embodiment of the pedometer with pulse count device in this invention;
Fig. 11 shows a flow chart of an action noise signal detecting procedure in a CPU in an embodiment of the pedometer with pulse count device;
Fig. 12 shows a flow chart of a graphically displaying procedure in a CPU of an embodiment of the pedometer with pulse count device in this invention; and
Fig. 13 shows a flow chart of a distance calculating and displaying procedure in a CPU of an embodiment of the pedometer with pulse count device in this invention.

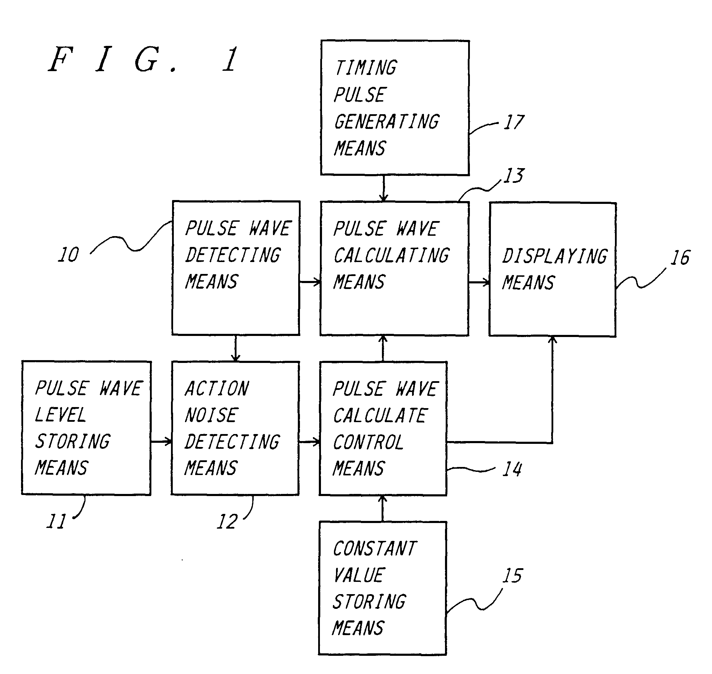

Fig. 1 shows a functional block diagram which represents an example of a typical constitution of this invention. The pulse wave detecting means 10 detects a pulse wave of a human body and outputs a pulse wave signal which has been detected to an action noise detecting means 12 and to a pulse wave calculating means 13. The action noise detecting means 12 outputs an action noise signal to a pulse wave calculate control means 14 by comparing the stored contents in a pulse wave level storing means 11 with the pulse wave signal which has been input. The pulse wave calculate control means 14, based on the action noise input to it, selects various constant values stored in a constant value storing means 15, and outputs the various constant values and the result of its selection to the pulse wave calculating means 13 and a displaying means 16. The pulse wave calculating means 13 calculates a result on the basis of the pulse wave signal received by it using a standard signal which a timing pulse generating means 17 outputs and the various constant values which the pulse wave calculate control means 14 outputs, and output the calculated result to the displaying means 16.

The pulse wave which the pulse wave detecting means 10 detects is output to not only the pulse wave calculating means 13 but also to the action noise detecting means 12 in case of the users being quiet when detecting the pulse or when detecting the step signal of his/her swinging an arm in case of his/her walking so as to be able to determine the step number.

The signal width level which is output from the pulse wave detecting means 10 is different from each other depending upon whether the user is keeping quiet or walking. The action noise detecting means 12 determines which of the signals is received, due to the stored level in the pulse wave level storing means 11. Accordingly, the displaying means 16 displays pulse wave information based on the resultant output of the pulse wave calculate control means 14 in case of the user keeping quiet, or step number information [as well as its identified signal 50 at the same time] in case of the user walking. The action noise detecting means 12 can detect not only walking but also jogging or running according to the stored level in the pulse wave level storing means 11.

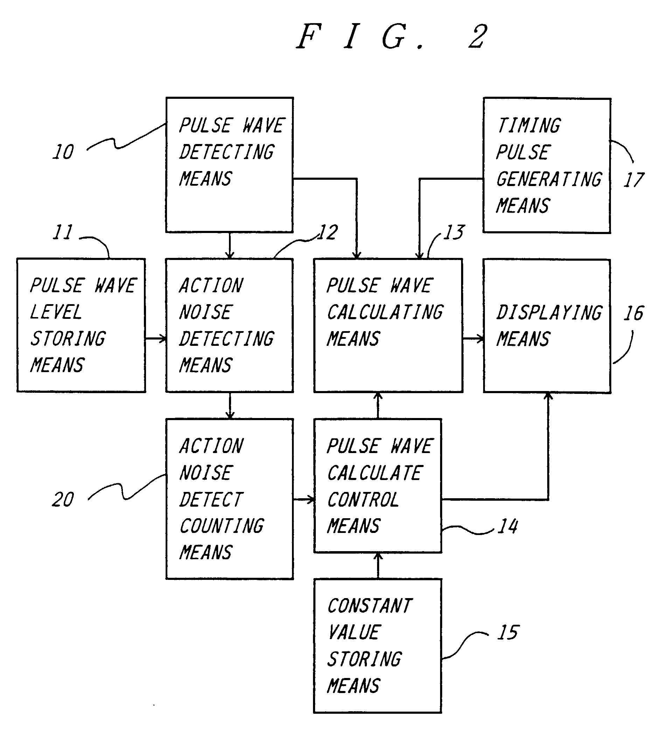

Fig. 2 shows a functional block diagram which represents another example of the typical constitution of this invention. The pulse wave detecting means 10 detects a pulse wave of a human body and outputs the detected pulse wave signal to the action noise detecting means 12 and to the pulse wave calculating means 13. The action noise detecting means 12 outputs the action noise signal to an action noise detect counting means 20 by comparing the pulse wave signal with the stored contents in the pulse wave level storing means 11. The action noise detect counting means 20 measures the duration in which the action noise input into it maintains the predetermined level, and outputs it as the action noise measuring signal to the pulse wave calculate control means 14.

The pulse wave calculate control means 14 selects, based on the action noise signal input to it, the various constant values stored in the constant value storing means 15, and outputs the various constant values and the selected result to the pulse wave calculating means 13 and to the displaying means 16. The pulse wave calculating means 13 calculates the result using the pulse wave signal input to it, the standard signal output by the timing pulse generating means 17 and the various constant values outputs by the pulse wave calculate control means 14, to output the result into the displaying means 16.

Since the action noise signal, which the action noise detect counting means 20 detects, is maintained, its action noise signal level is not affected by momentary or instant change in the action noise signal itself. Thus what is displayed on the displaying means 16, does not vary momentarily or with instance.

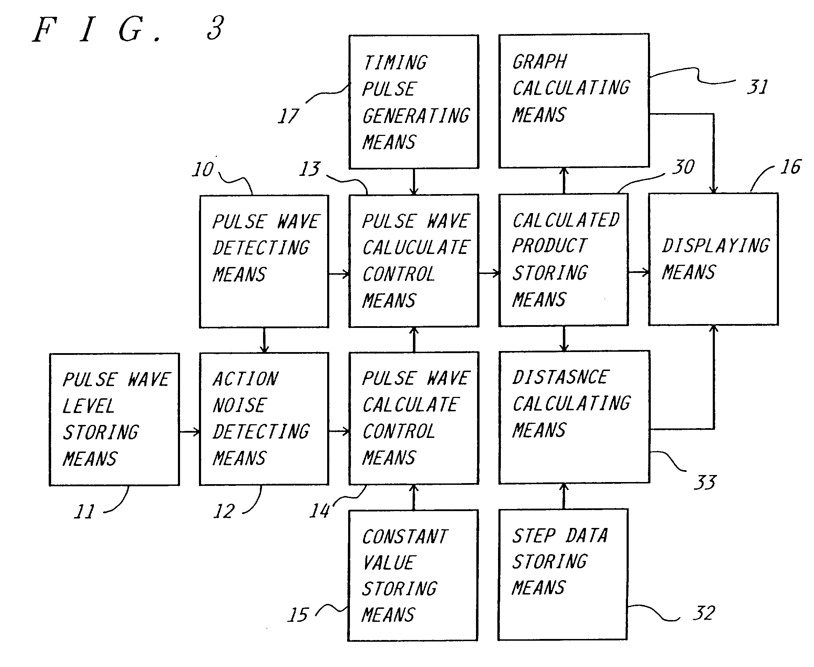

Fig. 3 shows a functional block diagram of another example of a typical constitution of this invention. The pulse wave detecting means 10 detects a pulse wave of a human body, and outputs its signal to the pulse wave calculating means 13 and to the action noise detecting means 12. The action noise detecting means 12 compares a pulse wave signal input to it, with the stored contents in the pulse wave level storing means 13, and outputs the action noise signal to the pulse wave calculated control means 14. The pulse wave calculates control means 14 selects, based on the action noise signal input to it, the various constant values stored in the constant value storing means 15, and outputs the various constant values to the pulse wave calculating means 13. The pulse wave calculating 13 calculates a result using the pulse wave signal input to it and both the standard signal output by the timing pulse generating means 17 and the various constant values output by the pulse wave calculate control means 14, and outputs the result to the calculated product storing means 30.

The calculated product storing means 30 not only stores the result but from time to time, also, if necessary, outputs the stored contents to the displaying means 16, a graph calculating means 31 and a distance calculating means 33. The graph calculating means 31 calculates data, using the calculated and stored contents input to it, for the purpose of a display time-series graph, and outputs the calculated result to the displaying means 16. The distance calculating means 33 calculates the distance which the user has walked using the calculated and stored contents of the calculated product storing means 30 and the stored contents in a step data storing means 32, outputs the calculated result to the displaying means 16.

By means of the graph calculating means 31 and the distance calculating means 33, not only the display of the pulse wave measuring function and the step number measuring function, but also the display of time series changes in the above can be realised and usage as a distance finder can be realised, too.

Embodiments of this invention are explained hereinafter based on the drawings.

### (1) The first embodiment

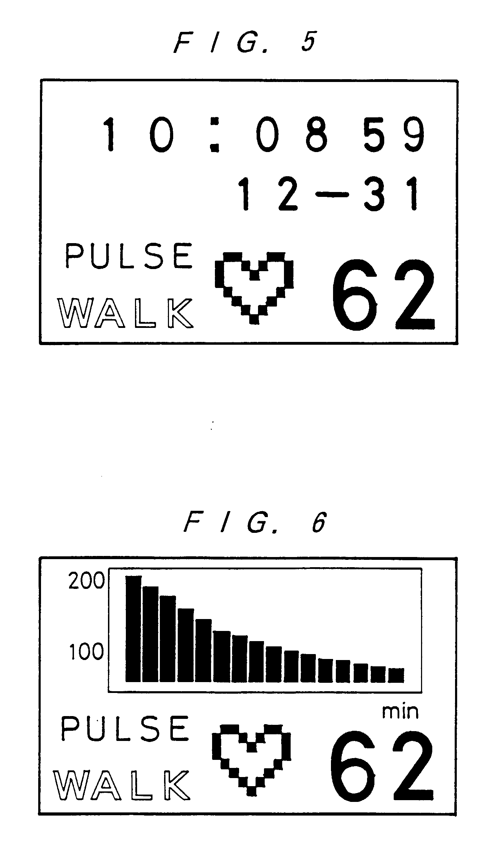

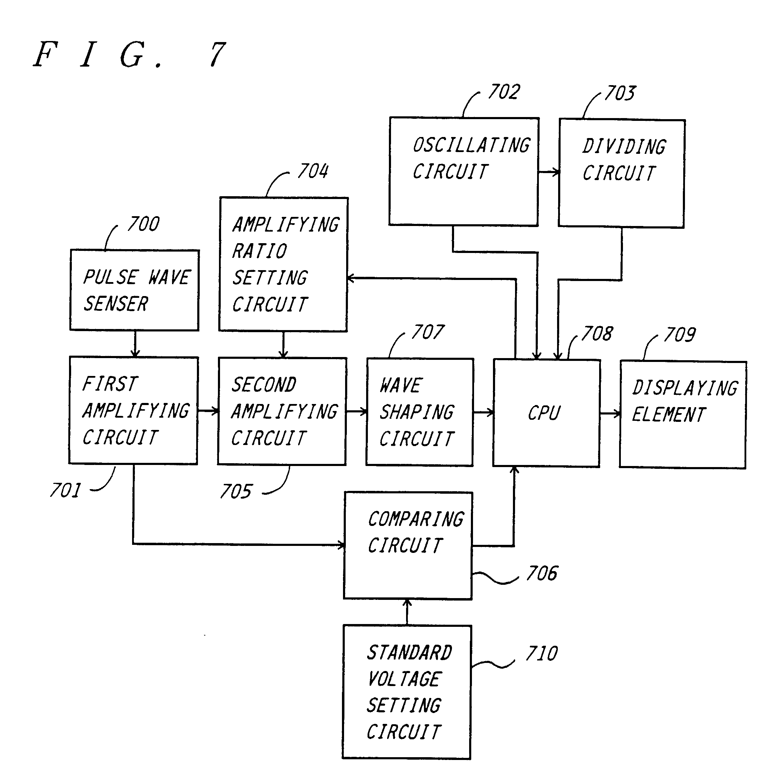

Fig. 7 shows a functional block diagram of the first embodiment of an electronic pedometer with pulse counting device of this invention. The pulse wave sensor 700 detects pulse waves of a human body, and output the pulse wave signal which has been detected to the first amplifying circuit 701. The first amplifying circuit 701 amplifies the pulse wave signal and outputs to the second amplifying circuit 705 and to the comparing circuit 706. The comparing circuit 706 compares the pulse wave signal level input to it with the electric voltage level standard stored in the standard voltage setting circuit 710 and outputs to a CPU 708 the compared result as the detected action noise signal. CPU 708 outputs, based on this comparison result above, an amplifying ratio setting signal to an amplifying ratio setting circuit 704 and outputs at the same time an identified signal 50 as shown in Fig. 5 to a displaying element 709. According to the identified signal 50, the user can identify instantly which datum is measured now in the system.

The amplifying ratio setting circuit 704 sets the signal amplifying ratio for the second amplifying circuit 705, based on the amplifying ratio setting signal. The second amplifying circuit 705 improves the S/N ratio of the pulse wave signal input to it by way of a filter circuit etc., and amplifies the pulse wave signal in accordance with the amplifying ratio set by the amplifying ratio setting circuit 704. The second amplifying circuit 705 then outputs the amplified pulse wave signal above to a wave shaping circuit 707. The wave shaping circuit 707 transforms the pulse wave signal which is input as an analogue signal into that which is a digital signal, and outputs the digital signal to the CPU 708. CPU 708 calculates the cycle of the pulse wave signal which is transformed to digital by way of the wave shaping circuit 707, based on the timing signal of a dividing circuit 703, and outputs the result to the displaying element, such as a liquid crystal panel etc.

Fig. 9 shows an embodiment of a concrete circuit of a functional block diagram showing in Fig. 7. The pulse wave sensor 700 detects changes in the blood stream by way of optical means. That is to say, a light intercepting element TR1, such as photo-transistor etc, detects transmitted light or reflected light from a luminescent element D1, such as an LED etc. For example, the sensor 700 is finger-tip shaped and is placed on a finger tip. Inside the pulse wave sensor 700 is equipped with a sensor holding component have a contact with a finger tip portion at predetermined pressure. This pulse wave sensor 700 can detect the blood stream state on the finger-tip.

Another pulse wave detecting means such as a microphone, can also detect the cardiac second as the pulsation. Like above, the pulse wave sensor 700 detects the pulse wave signal from a human body, and outputs it to the first amplifying circuit 701. The first amplifying circuit 701 rejects the direct current component from the pulse wave signal which it receives by way of filter. The filter comprises a condenser C1 and a resistor R3, and amplifiers it through a non-converted amplifier A1 by way of operational amplifier. The first amplifying circuit then outputs the pulse wave signal into the second amplifying circuit 705 and to the comparing circuit 706. The standard voltage setting circuit 710 sets the standard voltage by way of the resisters R13 and R14, and outputs the standard voltage to the comparing circuit 706.

The comparing circuit 706 comprises window-comparators A5 and A6 for the purpose of judging the width level of the pulse wave signal which is input. The width level is indicative of whether the holder is keeping quiet or whether he/she is swinging the arms. The judgement is done by comparing the pulse wave signal with the standard voltage set according to the resistive dividing ratio of resistors R13 and R14. In this case, the judgement is as follows: output from the gate circuit G1 is at VCC level in case of the user keeping quiet, and it is at GND level in case of the user swinging arms: i.e. in walking. If the amplifying ratio at the first amplifying circuit 701 and/or resistive dividing ratio at the standard voltage setting circuit 710 is changed, then as well as the user being detected as walking, one can also detect whether the user is running.

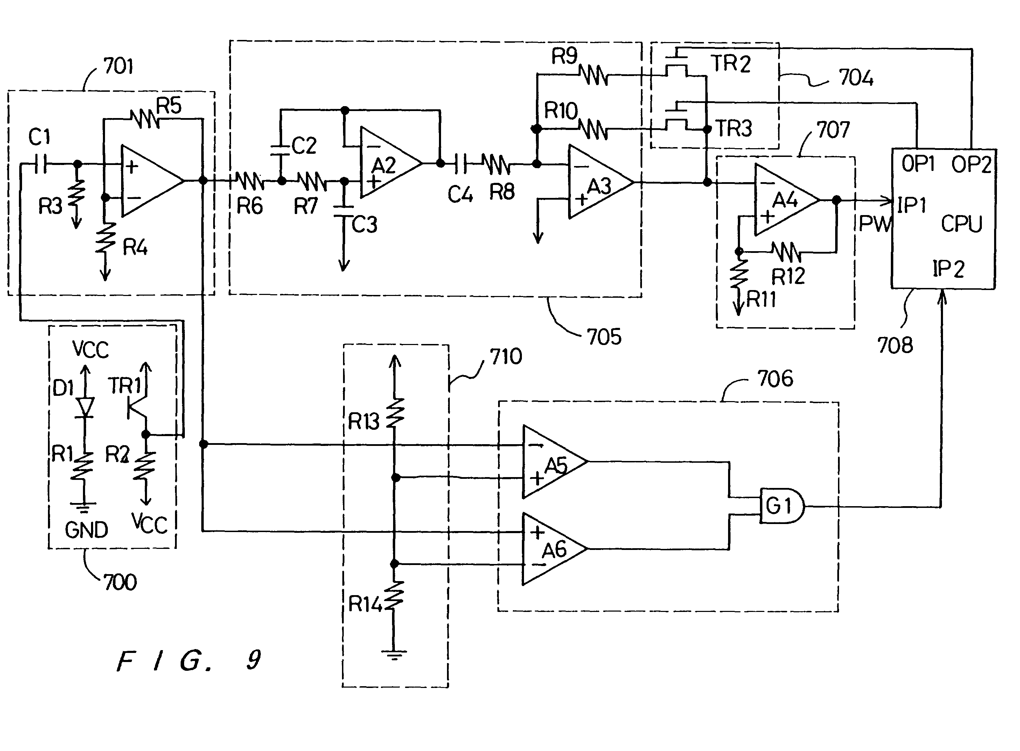

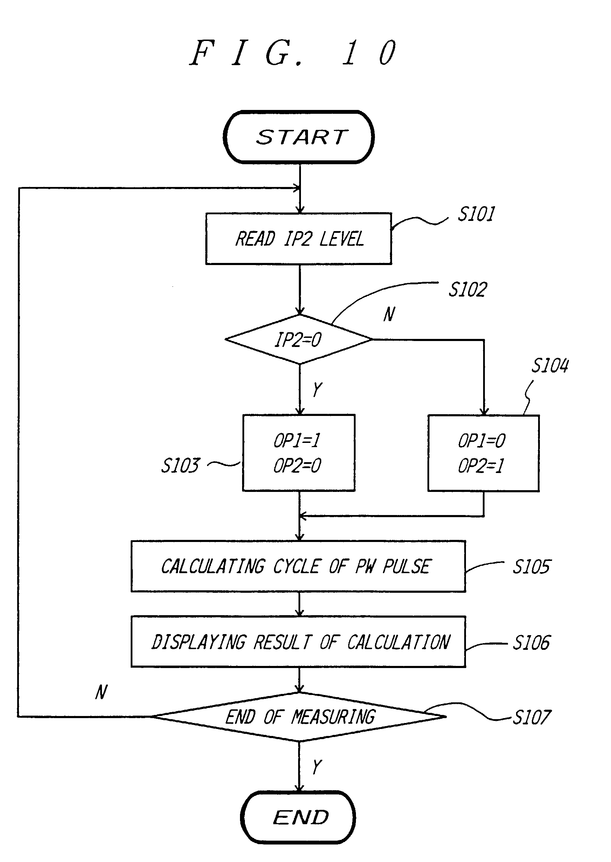

Fig. 10 shows a flow chart of a procedure when the CPU 708 controls the amplifying ratio setting circuit 704, based on the action noise signal P/W which is output from the comparing circuit 706, and when the pulse wave signal cycle is output to the displaying element. In Fig. 10 and Fig. 9, CPU 708 reads the signal level at input port IP2 terminal and judges its level (S101, S102). In case of GND level, the resistor R10 is selected by turning-on transistor TR3. In case of VCC level, the resistor R9 is selected by turning on transistor TR2 (S103, S104).

In Fig. 9, any noise component included in the pulse wave signal which is input to the second amplifying circuit 705, is rejected by the filter circuit which comprises of resistors R6, and R7, condensers C2 and C3, and operational amplifier A2. After the rejection above, the pulse wave signal is amplified by way of converted amplifier which comprises of operational amplifier A3 and resistors R8, R9 and R10. The pulse wave signal is converted to digital through wave shaping circuit 707 and then output to input port IP1 terminal of CPU 708 as PW pulse. At this occasion as shown in this embodiment, it is possible that noise is rejected adding a hysteresis characteristic by way of positive feedback of the operational amplifier output through resistor R12.

In Fig. 10, CPU 708 calculates the PW pulse cycle received based on a standard signal (S105). To calculate this, the time difference between the former PW pulse and current PW pulse may be measured relatively simply or alternatively the number of input durations of PW pulse to be measured may be also accepted. Any result above is displayed at the displaying element 709 as a number of signals for one minute (S106). After display, the measurement is continued if in measuring mode. Alternatively automatic stoppage of measurement after measuring for a certain period of time can also be arranged.

### (2) The second embodiment

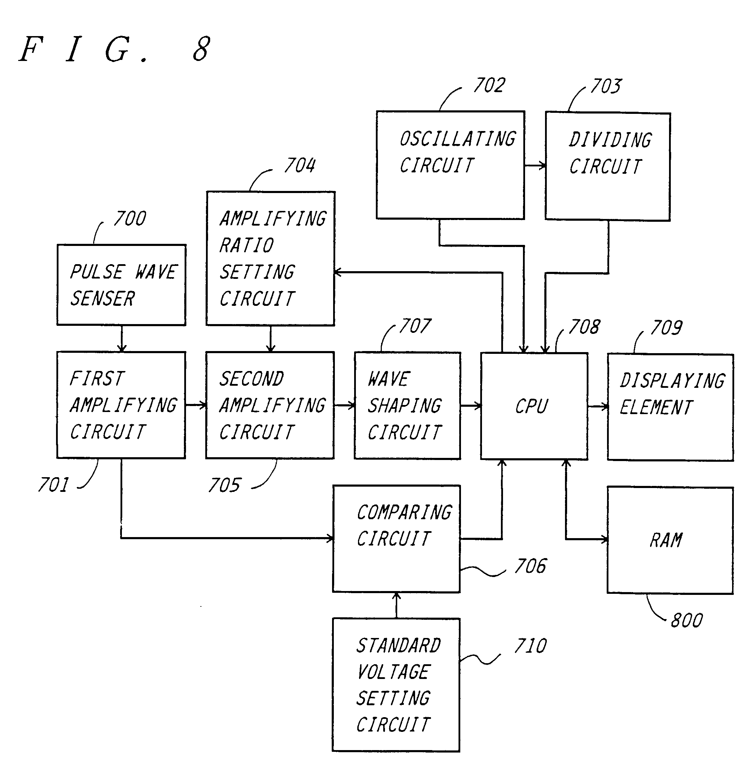

Fig. 8 shows a functional block diagram of the second embodiment of the electronic pedometer with pulse count device in this invention. In this embodiment, RAM 800 for datum memory is added. That is, RAM 800 is added to the pedometer with pulse count device shown in Fig. 7. RAM 800 is efficiently utilised so that the total memory area is divided to include, for example, PW pulse memory area, cycle calculating area, graphic datum memory area, step length memory area, and distance calculating area.

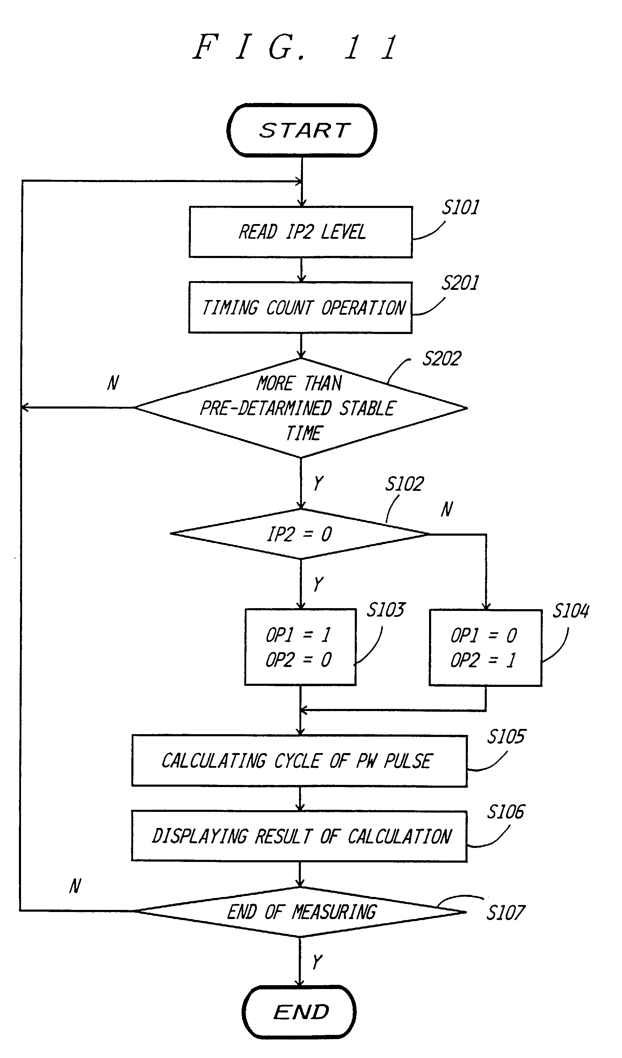

Fig. 11 shows a flow chart of the procedure which includes a stable duration of time during which action noise detecting signal P/W which is detected by comparing circuit 706, is measured so that the amplifying ratio setting circuit 704 is controlled by the CPU action noise detecting signal P/W read from input port IP2, and if its level is at the same level as of the former one, then a period of time is counted. The time is counted in the time counting area which is divided beforehand in RAM 800. If the input level is different from that of the former, then the content to be counted is reset and is counted again from the first (S201). If the counted number is greater than a predetermined one, then the calculating and displaying procedure is followed as shown in Fig. 10 (S202). Due to the above procedure, the displayed content is not instantly altered on momentary changes in action noise, so that the user can be confident of using the pedometer.

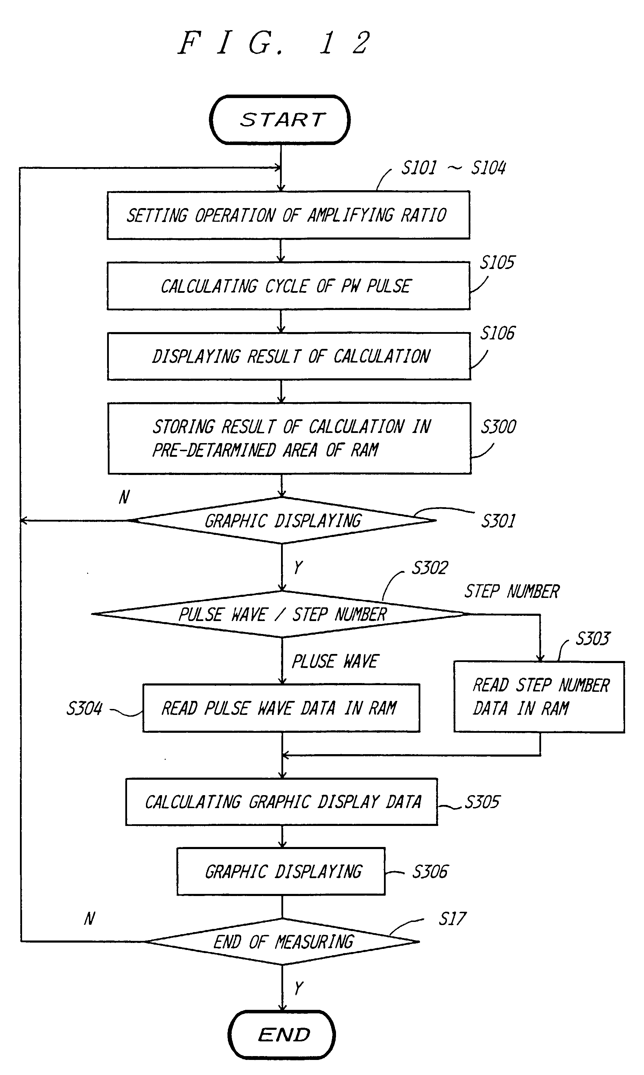

Fig. 12 shows a flowchart of the procedure for displaying time series graphically for instance, the cycle calculation result of the detected pulse wave signal. After the cycle calculation and displaying procedure shown in Fig. 10, the result above is stored in the PW pulse memory which is part of RAM 800 (S300). After this, it is judged whether the display is in graphically displaying mode or not (S301). If the display is in the graphically displaying mode, data stored in RAM 800 is converted to be displayable data in expected graph style after judging that the display is in the graphically displaying mode (S305). Then, for example as shown in Fig. 6, a time series graph is displayed (S306). By way of a change in the calculation procedure, any additionally flicker display of the smallest or the largest value may be done or alternatively the display may be swept so that display is not completed at once.

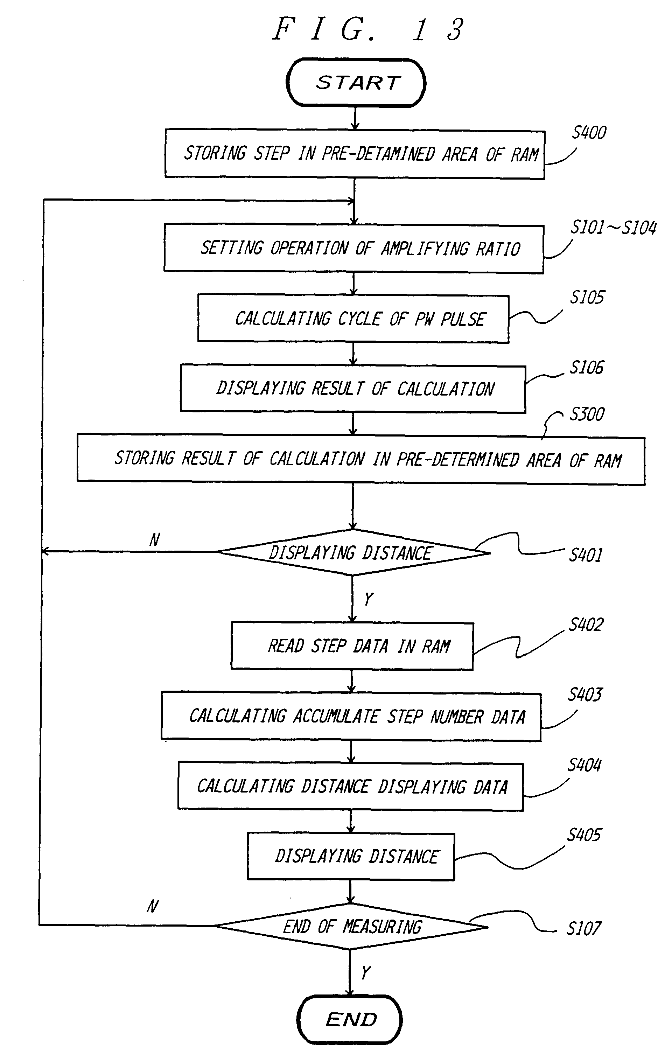

Fig. 13 shows a flow chart of the displaying procedure for the displaying element 709 by way of distance calculation utilising step width datum and the cumulative step number stored in RAM 800. At first beforehand, the step width is memorised in the step width datum memory area in RAM 800 (S300). Next, after cycle calculation and its displaying procedure shown in Fig. 10, the result of calculation is stored in PW pulse memory area divided in RAM 800 (S300). After judging that the display is in distance displaying mode (S401), the memory is read to obtain the step width datum in RAM 800 (S402) and the cumulative stem number stored in the other area in RAM 800 is calculated (S403). The result is displayed on the displaying element 709 (S405). Not only the distance but also the time series shift type display of cumulative distance (S404) can graphically displayed by way of graphical display procedure shown in Fig. 12.

As explained above, a sensor can be used combining pulse wave and step number counting in the electronic pedometer with pulse count device in this invention. This leads to the realisation of smaller sizing and compact, portable types of product. By changing the standard voltage for judging the signal level detected, not only walking but also running can also be measured (this usage is called a pitchmeter). Therefore various kinds of multipurpose devices as shown above can be offered as a consequence of this invention.

The aforegoing description has been given by way of example only and it will be appreciated by a person skilled in the art that modifications can be made without departing from the scope of the present invention, the scope of invention being defined in the appended claims 1-7.

## Claims

1. An electronic pedometer comprising:
a pulse wave detecting means (10;700) for detecting the pulse wave of a mammal; and
pulse wave calculating means (13;705;707;708) for amplifying an output signal from the pulse wave detecting means and for calculating the pulse rate and number of steps of the mammal;
characterised in that the pedometer further comprises pulse wave level storing means (11;710) for storing historic data correlating type of movement with pulse wave characteristics of mammals;
action noise detecting means (12;706) for comparing the signal received from the pulse wave detecting means with data stored in the pulse wave level storing means thereby detecting the type of movement of the mammal;
constant value storing means (15;708) for storing historic data of an amplifying factor of the pulse wave calculating means; and
pulse wave calculate control means (14;704;708) for selecting an amplifying factor of the pulse wave calculating means based on an output signal of the action noise detecting means and an output signal of the constant value storing means.

2. An electronic pedometer in accordance with claim 1 wherein the pedometer further comprises timing pulse generating means (17;703).

3. An electronic pedometer of any preceding claim wherein the pedometer further comprises a display means (16;709).

4. An electronic pedometer of any preceding claim wherein the pedometer further comprises:
action noise detect counting means (20; 705) for outputting a signal to the pulse wave calculate control means on detecting that the mammal has maintained a type of movement for a predetermined duration.

5. The electronic pedometer of claim 3 wherein the display means receives information from the action noise detecting means and displays the type of movement of the mammal.

6. An electronic pedometer of any preceding claim further comprising calculated product storing means (30) which stores information concerning a pulse wave which said pulse wave calculating means outputs; and
graph calculating means (31) for calculating data to be displayed as a time series graph on said display means.

7. An electronic pedometer of any preceding claim further comprising:
step data storing means (32) which stores steps;
distance calculating means (33) which calculates distance which is walked through the steps above; and
said display means which displays the distance by receiving distance calculating signal which is output by said distance calculating means.

## Patentansprüche

1. Elektronischer Schrittmesser, umfassend:
- ein Pulswellenerfassungsmittel (10; 700) zum Erfassen der Pulswelle eines Säugers und
- ein Pulswellenberechnungsmittel (13; 705; 707; 708) zum Verstärken eines Ausgangssignals des Pulswellenerfassungsmittels und zum Berechnen der Pulsrate und der Schrittzahl des Säugers,
**dadurch gekennzeichnet**, daß der Schrittmesser ferner umfaßt:
- ein Pulswellenpegelspeichermittel (11; 710) zum Speichern historischer Daten, welche eine Bewegungsart mit Pulswelleneigenschaften von Säugern korrelieren,
- ein Aktionsrauscherfassungsmittel (12; 706) zum Vergleichen des von dem Pulswellenerfassungsmittel erhaltenen Signals mit in dem Pulswellenpegelspeichermittel gespeicherten Daten, um hierdurch die Bewegungsart des Säugers zu erfassen,
- ein Konstantwertspeichermittel (15; 708) zum Speichern historischer Daten eines Verstärkungsfaktors des Pulswellenberechnungsmittels und
- ein Pulswellenberechnungssteuermittel (14; 704; 708) zum Wählen eines Verstärkungsfaktors des Pulswellenberechnungsmittels auf Grundlage eines Ausgangssignals des Aktionsrauscherfassungsmittels und eines Ausgangssignals des Konstantwertspeichermittels.

2. Elektronischer Schrittmesser nach Anspruch 1, bei dem der Schrittmesser ferner ein Taktimpulserzeugungsmittel (17; 703) umfaßt.

3. Elektronischer Schrittmesser nach einem der vorhergehenden Ansprüche, bei dem der Schrittmesser ferner ein Anzeigemittel (16; 709) umfaßt.

4. Elektronischer Schrittmesser nach einem der vorhergehenden Ansprüche, bei dem der Schrittmesser ferner ein Aktionsrauscherfassungszählmittel (20; 705) umfaßt, um dann, wenn erfaßt wird, daß der Säuger eine Bewegungsart für eine vorbestimmte Dauer beibehalten hat, ein Signal an das Pulswellenberechnungssteuermittel auszugeben.

5. Elektronischer Schrittmesser nach Anspruch 3, bei dem das Anzeigemittel Informationen von dem Aktionsrauscherfassungsmittel erhält und die Bewegungsart des Säugers anzeigt.

6. Elektronischer Schrittmesser nach einem der vorhergehenden Ansprüche, ferner umfassend
- ein Berechnungsproduktspeichermittel (30), welches Informationen speichert, die eine Pulswelle betreffen, welche das Pulswellenberechnungsmittel ausgibt, und
- ein Graphikberechnungsmittel (31) zum Berechnen von Daten, die als Zeitreihengraphik auf dem Anzeigemittel anzuzeigen sind.

7. Elektronischer Schrittmesser nach einem der vorhergehenden Ansprüche, ferner umfassend:
- ein Schrittdatenspeichermittel (32), das Schritte speichert,
- ein Streckenberechnungsmittel (33), das eine Strecke berechnet, welche durch die obigen Schritte zurückgelegt wird, und
- ein Anzeigemittel, das die Strecke anzeigt, indem es ein Streckenberechnungssignal erhält, welches von dem Streckenberechnungsmittel ausgegeben wird.

## Revendications

1. Pédomètre électronique comprenant :
des moyens de détection d'onde d'impulsion (10 ; 700) destinés à détecter l'onde d'impulsion d'un mammifère et
des moyens de calcul d'onde d'impulsion (13 ; 705 ; 707 ; 708) destinés à amplifier un signal de sortie provenant des moyens de détection d'onde d'impulsion et destinés à calculer la fréquence des impulsions et le nombre de pas du mammifère ;
caractérisé en ce que le pédomètre comprend, de plus, des moyens de mémorisation de niveau d'onde d'impulsion (11 ; 710) destinés à mémoriser des données d'historique mettant en corrélation un type de mouvement avec des caractéristiques d'onde d'impulsion de mammifères ;
des moyens de détection de bruit d'action (12 ; 706) destinés à comparer le signal reçu à partir des moyens de détection d'onde d'impulsion avec les données mémorisées dans les moyens de mémorisation de niveau d'onde d'impulsion, détectant de ce fait le type de mouvement du mammifère
des moyens de mémorisation de valeur constante (15 708) destinés à mémoriser des données d'historique d'un coefficient d'amplification des moyens de calcul d'onde d'impulsion ; et
des moyens de contrôle de calcul d'onde d'impulsion (14 ; 704 ; 708) destinés à sélectionner un coefficient d'amplification des moyens de calcul d'onde d'impulsion sur la base d'un signal de sortie des moyens de détection de bruit d'action et d'un signal de sortie des moyens de mémorisation de valeur constante.

2. Pédomètre électronique selon la revendication 1, comprenant en outre des moyens de génération d'impulsions de synchronisation (17 ; 703).

3. Pédomètre électronique selon l'une quelconque des revendications précédentes, comprenant en outre des moyens d'affichage (16 ; 709).

4. Pédomètre électronique selon l'une quelconque des revendications précédentes, comprenant en outre :
des moyens de comptage de détection de bruit d'action (20 ; 705) destinés à sortir un signal vers les moyens de contrôle de calcul d'onde d'impulsion lors de la détection du fait que le mammifère a maintenu un type de mouvement pendant une durée prédéterminée.

5. Pédomètre électronique selon la revendication 3, dans lequel les moyens d'affichage reçoivent des informations à partir des moyens de détection de bruit d'action et affichent le type de mouvement du mammifère.

6. Pédomètre électronique selon l'une quelconque des revendications précédentes comprenant, de plus, des moyens de mémorisation de produit calculé (30) qui mémorisent des informations concernant une onde d'impulsion sortie par lesdits moyens de calcul d'onde d'impulsion ; et
des moyens de calcul de graphique (31) destinés à calculer des données à afficher en tant que graphique de série temporelle sur lesdits moyens d'affichage.

7. Pédomètre électronique selon l'une quelconque des revendications précédentes comprenant de plus :
des moyens de mémorisation de données de pas (32) qui mémorisent les pas ;
des moyens de calcul de distance (33) qui calculent la distance parcourue par les pas ci-dessus ; et
lesdits moyens d'affichage qui affichent la distance en recevant le signal de calcul de distance qui est sorti par lesdits moyens de calcul de distance.
